# EUROPEAN PATENT APPLICATION

(11) **EP 4 570 819 A1**
(43) Date of publication of application: **18.06.2025**
(21) Application number: 23851744.5
(22) Date of filing: 07.08.2023
(51) Int. Cl.: C07K 14/605

(54) **LONG-ACTING DUAL-AGONIST COMPOUND**

(30) Priority: 10.08.2022 CN 202210957875
(71) Applicant: Chengdu Aoda Biotechnology Co., Ltd., Chengdu, Sichuan 610041 (CN)
(72) Inventor: ZHOU, Shuliang, Chengdu, Sichuan 610041 (CN); WANG, Peng, Chengdu, Sichuan 610041 (CN); DENG, Lan, Chengdu, Sichuan 610041 (CN)
(74) Representative: PGA S.p.A., Milano, Succursale di Lugano
(86) International application number: PCT/CN2023/111384
(87) International publication number: WO 2024/032523

(57) **Abstract**

The present invention relates to the field of pharmaceutical synthesis. A GLP-1/Gcg dual-agonist compound is disclosed. The GLP-1/Gcg dual-agonist compound is used for preparing a pharmaceutical composition for treating disease. Use of the pharmaceutical composition in the preparation of a drug for treating at least one of the following diseases: type-II diabetes, impaired glucose tolerance, type-I diabetes, obesity, hypertension, metabolic syndrome, dyslipidemia, cognitive disorders, atherosclerosis, myocardial infarction, coronary heart disease, cardiovascular disease, strokes, inflammatory bowel syndrome and/or dyspepsia or gastric ulcers, hepatic fibrosis disease, and pulmonary fibrosis disease.

## Description

This application claims the priority of Chinese Patent Application No. 202210957875.5 filed with the China National Intellectual Property Administration on August 10, 2022, and titled with "LONG-ACTING DUAL-AGONIST COMPOUND", the disclosure of which is hereby incorporated by reference in its entirety.

### FIELD

The present invention relates to the field of pharmaceutical synthesis, in particular to a long-acting dual-agonist compound, which is a dual-agonist compound of glucagon (Gcg) receptor and glucagon-like peptide-1 (GLP-1) receptor.

### BACKGROUND

GLP-1 is a 37-amino-acid peptide that stimulates insulin secretion, protects pancreatic beta cells, and inhibits glucagon secretion, gastric emptying and food intake, leading to weight loss. GLP-1 is known as an incretin. Incretin receptor signaling plays a key physiologically relevant role in glucose homeostasis. In normal physiology, GLP-1 is secreted from the intestine after a meal. These incretins enhance physiological responses to food, including satiety, insulin secretion, and nutrient disposal.

The most common side effect of GLP-1 analogs is that the administration thereof does not achieve full-effect blood sugar control and weight loss, while GIP alone has a very modest glucose-lowering ability in patients with type 2 diabetes. Natural GLP-1 can be rapidly inactivated by the ubiquitous protease DPP IV and can therefore only be used for short-term metabolic control.

Gcg, also known as glucagon or anti-insulin or insulin B, is a hormone secreted by the pancreatic islet alpha cells of the vertebrate pancreas along with insulin, which antagonizes insulin and plays a role in increasing blood sugar.

New studies have shown that dual GLP-1 and Gcg receptor agonists not only have better blood sugar control, but also have the effect of significantly reducing weight and treating non-alcoholic fatty liver disease.

Currently, there is no dual GLP-1/Gcg receptor agonist on the market. The half-life of the dual GLP-1/Gcg receptor agonist OXM3 in clinical research is 120 hrs, and the administration cycle is once a week.

### SUMMARY

In view of this, the present invention provides a long-acting dual agonist compound, which is a dual agonist compound of glucagon (Gcg) receptor and glucagon-like peptide-1 (GLP-1) receptor.

In order to achieve the above-mentioned invention purpose, the present invention provides the following technical solution.

The present invention provides a compound, comprising:
(I) an amino acid sequence set forth in Formula I,

   His-AA1-Gln-Gly-Thr-Phe-Thr-Ser-Asp-Tyr-Ser-Lys-Tyr-Leu-Asp-Glu-Lys-Lys-Ala-Lys(R)-Glu -Phe-Val-Glu-Trp-Leu-Leu-AA2-Gly-Gly-Pro-Ser-Ser-Gly-Ala-Pro-Pro-Pro-Ser-AA3 Formula I

   AA1 in Formula I is selected from the group consisting of Aib, Acpr, Acp, Acpe and Ach;
   AA2 in Formula I is selected from the group consisting of Glu and Ser;
   AA3 in Formula I is selected from the group consisting of NH₂ and OH;
   R in Formula I is selected from the group consisting of HO₂C(CH₂)ₙ₁CO-(AA4)ₙ₂-(PEGₙ₃(CH2)ₙ₄CO)ₙ₅- and HO₂C(CH₂)ₙ₁CO-(AA4)ₙ₂-(AA5)ₙ₆-;
   wherein, AA4 is selected from the group consisting of γGlu, εLys, β-Ala, γ-aminobutyric acid and 5-Ava; and
   AA5 is selected from the group consisting of Gly, Ser, Thr, Asp, Glu, Aad, Lys, Orn, Dab and Dap; or
(II) an amino acid sequence obtained from substitution, deletion, addition and/or replacement of one or more amino acids of the amino acid sequence set forth in (I) ; or
(III) a sequence with more than 90% homology to the amino acid sequence set forth in (I); or
(IV) a pharmaceutically acceptable salt, solvate, chelate or non-covalent complex of the compound shown in Formula I; and/or
(V) a drug precursor based on the compound shown in Formula I; and/or
(VI) a mixture comprising (I), (II), (III), (IV) and/or (V).

In some specific embodiments of the present invention,
n1 is an integer selected from 10 to 20;
n2 is an integer selected from 1 to 5;
n3 is an integer selected from 1 to 30;
n4 is an integer selected from 1 to 5;
n5 is an integer selected from 1 to 10; and
n6 is an integer selected from 1 to 10.

The present invention further provides a method for producing the compound, comprising:
Step 1, performing solid-phase polypeptide synthesis to obtain a peptide resin; and
Step 2, performing acid hydrolysis and purification to obtain the compound.

The present invention further provides use of any one of the following in the manufacture of a dual GLP-1 receptor and Gcg receptor agonist:
(I) the compound; and/or
(II) the compound produced by the method.

The present invention further provides use of any one of the following in the manufacture of a medicament for preventing and/or treating a disease:
(I) the compound; and/or
(II) the compound produced by the method.

In some specific embodiments of the present invention, the disease includes: type II diabetes, impaired glucose tolerance, type I diabetes, obesity, hypertension, metabolic syndrome, dyslipidemia, cognitive impairment, atherosclerosis, myocardial infarction, coronary heart disease, cardiovascular disease, stroke, inflammatory bowel syndrome and/or dyspepsia or gastric ulcer, liver fibrosis and/or pulmonary fibrosis.

The present invention further provides use of any one of the following in the manufacture of a drug and/or drug combination for treating type II diabetes with long-lasting efficacy and/or preventing deterioration of type II diabetes:
(I) the compound; and/or
(II) the compound produced by the method.

The present invention further provides use of the compound or the compound produced by the method in the manufacture of a drug and/or a drug combination for regulating blood sugar in the body;

wherein, the regulation of blood sugar in the body includes reducing food intake, reducing β cell apoptosis, increasing pancreatic β cell function, increasing β-cell mass and/or restoring the sensitivity of β-cell to glucose.

The present invention further provides a drug or drug combination, comprising any one of the following:
(I) the compound; and/or
(II) the compound produced by the method.

The present invention further provides a method for regulating blood sugar in the body, comprising administering any one of the following to a subject in need thereof:
(I) the compound; and/or
(II) the compound produced by the method; and/or
(III) the drug or drug combination.

The present invention includes but is not limited to the following beneficial effects.

The compound shown in Formula I provided by the present invention is a dual Gcg/GLP-I receptor agonist analog, which has the biological activity of lowering blood sugar and reducing body weight.

### DETAILED DESCRIPTION

The present invention discloses a long-acting dual agonist compound, and those skilled in the art can refer to the content herein and appropriately improve the process parameters to achieve it. It should be particularly noted that all similar substitutions and modifications are obvious to those skilled in the art, which are all considered to be included in the present invention. The method and application of the present invention have been described through preferred embodiments, and relevant personnel can obviously modify or appropriately change and combine the methods and applications described herein without departing from the content, spirit and scope of the present invention to achieve and apply the technology of the present invention.

The purpose of the present invention is to provide a dual GLP-1/Gcg receptor agonist compound with a longer half-life, so as to achieve the purpose of dosing once every 2 weeks or once every 4 weeks.

The present invention provides a long-acting dual agonist compound and use thereof, which is a dual agonist compound of glucagon-like peptide-1 (GLP-1) receptor and glucagon (Gcg) receptor.

To achieve the above purpose, the present invention first provides a compound shown in formula I, a pharmaceutically acceptable salt, solvate, chelate, non-covalent complex or a drug precursor thereof, or any mixture of the above forms.

His-AA1-Gln-Gly- Thr-Phe- Thr-Ser-Asp- Tyr-Ser-Lys- Tyr-Leu-Asp-Glu-Lys-Lys-Ala-Lys(R)-Glu -Phe-Val-Glu-Trp-Leu-Leu-AA2-Gly-Gly-Pro-Ser-Ser-Gly-Ala-Pro-Pro-Pro-Ser-AA3 Formula I

AA1 in Formula I is selected from the group consisting of Aib, Acpr, Acp, Acpe and Ach;
AA2 in Formula I is selected from the group consisting of Glu and Ser;
AA3 in Formula I is selected from the group consisting of NH₂ and OH;
R in Formula I is selected from the group consisting of HO₂C(CH₂)ₙ₁CO-(AA4)ₙ₂-(PEGₙ₃(CH2)ₙ₄CO)ₙ₅- and HO₂C(CH₂)ₙ₁CO-(AA4)ₙ₂-(AA5)ₙ₆-;
wherein: n1 is an integer selected from 10 to 20;
n2 is an integer selected from 1 to 5;
n3 is an integer selected from 1 to 30;
n4 is an integer selected from 1 to 5;
n5 is an integer selected from 1 to 10; and
n6 is an integer selected from 1 to 10;
AA4 is selected from the group consisting of γGlu, εLys, β-Ala, γ-aminobutyric acid and 5-Ava; and
AA5 is selected from the group consisting of Gly, Ser, Thr, Asp, Glu, Aad, Lys, Orn, Dab and Dap.

The present invention further provides a pharmaceutical composition comprising the compound according to the present invention, and provides use of the pharmaceutical composition comprising the compound according to the present invention in the manufacture of a medicament for treating a disease.

Preferably, the disease includes type II diabetes, impaired glucose tolerance, type I diabetes, obesity, hypertension, metabolic syndrome, dyslipidemia, cognitive impairment, atherosclerosis, myocardial infarction, coronary heart disease, cardiovascular disease, stroke, inflammatory bowel syndrome and/or dyspepsia or gastric ulcer, liver fibrosis and/or pulmonary fibrosis.

Preferably, the present invention provides use of the pharmaceutical composition in the manufacture of a medicament for treating type II diabetes with long-lasting efficacy and/or preventing deterioration of type II diabetes.

Preferably, the present invention provides use of the pharmaceutical composition in the manufacture of a medicament for reducing food intake, reducing β cell apoptosis, increasing pancreatic β cell function, increasing β-cell mass and/or restoring glucose sensitivity of β-cells.

The present invention further provides a method for regulating blood sugar in the body, comprising administering the compound to a subject in need thereof.

More contents of the present invention are described in detail below, or some can also be understood from the embodiments of the present invention.

Unless otherwise specified, the quantities of different components and reaction conditions used herein could be interpreted as "approximately" or "about" in any case. Accordingly, unless otherwise specified, the numerical parameters cited below and in the claims are approximate parameters, and different numerical parameters may be obtained under respective experimental conditions due to different standard errors.

**In** the present application, in case there is a disagreement or ambiguity between the chemical formula and the chemical name of a compound, the chemical formula is used to accurately define the compound. The compounds described herein may contain one or more chiral centers, and/or double bonds and the like, and may also exist as stereoisomers, including double bond isomers (such as geometric isomers), optical enantiomers or diastereomers. Accordingly, any chemical structure within the scope of the description herein, whether it contains the above-mentioned similar structure in part or in its entirety, includes all possible enantiomers and diastereomers of the compound, including any single stereoisomer (such as a single geometric isomer, a single enantiomer or a single diastereomer) and any mixture of these isomers. These mixtures of racemic isomers and stereoisomers can also be further separated into their constituent enantiomers or stereoisomers by those skilled in the art using different resolution techniques or chiral molecular synthesis methods.

The compounds of Formula I include, but are not limited to, optical isomers, racemates and/or other mixtures of these compounds. **In** the above case, a single enantiomer or diastereomer, such as an optically active isomer, can be obtained by asymmetric synthesis or racemate resolution. The resolution of the racemate can be achieved by various methods, such as conventional recrystallization with a reagent that assists the resolution, or by chromatography. **In** addition, the compounds of Formula I also include cis and/or trans isomers with double bonds.

The compounds described in the present invention include, but are not limited to, the compound represented by Formula I and all of the various pharmaceutically acceptable forms thereof, including various pharmaceutically acceptable salts, solvates, complexes, chelates, non-covalent complexes, prodrugs based on the above substances, and any mixture of the above forms.

The compounds represented by Formula I provided by the present invention are stable and are not easily degraded by dipeptidyl peptidase IV (DPP-IV) in the body. They function as Gcg/GLP-I dual agonist analogues with significant effect on lowering blood sugar and reducing body weight.

The present invention further provides a production method, comprising: taking Rink Amide MBHA resin as a starting resin, producing a peptide resin using solid phase peptide synthesis method, subjecting the peptide resin to acid hydrolysis to obtain a crude product, and finally purifying the crude product to obtain a pure product; wherein the step of producing a peptide resin using solid phase peptide synthesis method is performed by sequentially connecting the corresponding protected amino acids or fragments in the sequence to the carrier resin using solid phase coupling synthesis method to obtain the peptide resin.

In the above production method, the amount of the Fmoc-protected amino acid or protected amino acid fragment is 1.2 to 6 times the total molar number of the resin in the reaction; preferably 2.5 to 3.5 times.

In the above production method, the resin substitution is 0.2 to 1.0 mmol/g resin, preferably, the resin substitution is 0.3 to 0.5 mmol/g resin.

As a preferred embodiment of the present invention, the solid phase coupling synthesis method is performed by removing the Fmoc protecting group from the protected amino acid-resin obtained in the previous step, and then coupling the resulting product with the next protected amino acid; wherein, the removing Fmoc protecting group is performed for 10 to 60 minutes, preferably 15 to 25 minutes; and the coupling is performed for 60 to 300 minutes, preferably 100 to 140 minutes.

A condensation reagent is added in the coupling reaction, which is selected from the group consisting of DIC (N,N-diisopropylcarbodiimide), N,N-dicyclohexylcarbodiimide, benzotriazole-1-yl-oxytripyrrolidinophosphine hexafluorophosphate, 2-(7-aza-1H-benzotriazole-1-yl)-1,1,3,3-tetramethyluronium hexafluorophosphate, benzotriazole-N,N,N',N'-tetramethyluronium hexafluorophosphate and O-benzotriazole-N,N,N',N'-tetramethyluronium tetrafluoroborate, preferably N,N-diisopropylcarbodiimide; wherein, the molar number of the condensation reagent is 1.2 to 6 times the total molar number of amino groups in the amino resin, preferably 2.5 to 3.5 times.

An activation reagent is added in the coupling reaction, which is selected from the group consisting of 1-hydroxybenzotriazole and N-hydroxy-7-azabenzotriazole, preferably 1-hydroxybenzotriazole; wherein, the amount of the activation reagent is 1.2 to 6 times the total molar number of amino groups in the amino resin, preferably 2.5 to 3.5 times.

As a preferred embodiment of the present invention, the Fmoc-removing reagent is a mixed solution of PIP/DMF (piperidine/N,N-dimethylformamide), and the piperidine content in the mixed solution is 10 to 30% (V); the amount of the Fmoc-removing reagent is 5 to 15 mL per gram of amino resin, preferably 8 to 12 mL per gram of amino resin.

Preferably, the peptide resin is subjected to acid hydrolysis to remove the resin and side chain protecting groups to obtain a crude product.

Further preferably, the acid hydrolysis agent used in the acid hydrolysis of peptide resin is a mixed solvent of trifluoroacetic acid (TFA), 1,2-ethanedithiol (EDT) and water; and the volume ratio of the mixed solvent is 80 to 95% of TFA, 1 to 10% of EDT and water for the balance.

Further preferably, the volume ratio of the mixed solvent is 89 to 91% of TFA, 4 to 6% of EDT and water for the balance. Optimally, the volume ratio of the mixed solvent is 90% of TFA, 5% of EDT and water for the balance.

The amount of the acid hydrolysis agent used is 4 to 15 mL of acid hydrolysis agent per gram of peptide resin; preferably, the amount of acid hydrolysis agent used is 7 to 10 mL per gram of peptide resin.

The acid hydrolysis is performed for 1 to 6 hours at room temperature, preferably 3 to 4 hours.

Further, the crude product is purified by high performance liquid chromatography and freeze-dried to obtain a pure product.

The terms corresponding to the abbreviations involved in the present invention are shown in Table 1:

**Table 1 Comparison of terms and abbreviations**

| Abbreviation | Term | Abbreviation | Term |
|---|---|---|---|
| Fmoc | 9-Fluorenylmethox ycarbonyl | OtBu | tert-Butoxy |
| tBu | tert-Butyl | Boc | tert-Butoxycarbonyl |
| Trt | Trityl | Pbf | (2,3-dihydro-2,2,4,6,7-pe ntamethylbenzofuran-5-y l)sulfonyl |
| Ala | Alanine | Leu | Leucine |
| Arg | Arginine | Lys | Lysine |
| Asn | Asparagine | Met | Methionine |
| Asp | Aspartic acid | Phe | Phenylalanine |
| Cys | Cysteine | Pro | Proline |
| Gln | Glutamine | Ser | Serine |
| Glu | Glutamic acid | Thr | Threonine |
| Gly | Glycine | Trp | Tryptophan |
| His | Histidine | Tyr | Tyrosine |
| Ile | Isoleucine | Val | Valine |

The raw materials and reagents used in the production of long-acting dual agonist compound provided by the present invention are all commercially available.

The present invention is further described below in conjunction with examples:

### Example 1 Production of Compound 1

The sequence of Compound 1 is set forth in SEQ ID NO: 1:

His-Aib-Gln-Gly-Thr-Phe-Thr-Ser-Asp-Tyr-Ser-Lys-Tyr-Leu-Asp-Glu-Lys-Lys-Ala-Lys(AEEA-
AEEA-γGlu-eicosanedioic acid)-Glu-Phe-Val-Glu-Trp-Leu-Leu-Glu-Gly-Gly-Pro-Ser-Ser-
Gly-Ala-Pro-Pro-Pro-Ser-NH2

### 1. Synthesis of peptide resin

Rink Amide BHHA resin was used as a carrier resin, and the protected amino acids shown in the following table were sequentially connected to the resin through removing Fmoc protection and coupling reaction to obtain a peptide resin. The protected amino acids used in this example are shown in Table 2:

**Table 2 Protected amino acids used in Example 1**

| Order of connecting peptide n= | Protected amino acid |
|---|---|
| 1 | Fmoc-Ser(tBu) |
| 2 | Fmoc-Pro |
| 3 | Fmoc-Pro |
| 4 | Fmoc-Pro |
| 5 | Fmoc-Ala |
| 6 | Fmoc-Gly |
| 7 | Fmoc-Ser(tBu) |
| 8 | Fmoc-Ser(tBu) |
| 9 | Fmoc-Pro |
| 10 | Fmoc-Gly |
| 11 | Fmoc-Gly |
| 12 | Fmoc-Glu(OtBu) |
| 13 | Fmoc-Leu |
| 14 | Fmoc-Leu |
| 15 | Fmoc-Trp(Boc) |
| 16 | Fmoc-Glu(OtBu) |
| 17 | Fmoc-Val |
| 18 | Fmoc-Phe |
| 19 | Fmoc-Glu(OtBu) |
| 20 | Fmoc-Lys(Alloc) |
| 21 | Fmoc-Ala |
| 22 | Fmoc-Lys(Boc) |
| 23 | Fmoc-Lys(Boc) |
| 24 | Fmoc-Glu(OtBu) |
| 25 | Fmoc-Asp(OtBu) |
| 26 | Fmoc-Leu |
| 27 | Fmoc-Tyr(tBu) |
| 28 | Fmoc-Lys(Boc) |
| 29 | Fmoc-Ser(tBu) |
| 30 | Fmoc-Tyr(tBu) |
| 31 | Fmoc-Asp(OtBu) |
| 32 | Fmoc-Ser(tBu) |
| 33 | Fmoc-Thr(tBu) |
| 34 | Boc-Phe |
| 35 | Fmoc-Thr(tBu) |
| 36 | Fmoc-Gly |
| 37 | Fmoc-Gln(Trt) |
| 38 | Fmoc-Aib |
| 39 | Boc-His(Trt) |
| Side chain-1 | Fmoc-AEEA |
| Side chain-2 | Fmoc-AEEA |
| Side chain-3 | Fmoc-γGlu-OtBu |
| Side chain-4 | 20-(tert-Butoxy)-20-oxoicosanoic acid |

### (1) Coupling of the first protected amino acid in the main chain

3 mmol of the first protected amino acid and 3 mmol of HOBt were dissolved in an appropriate amount of DMF; 3 mmol of DIC was slowly added into the solution of protected amino acid in DMF under stirring, and the mixture was subjected to reaction at room temperature for 30 min with stirring to obtain an activated protected amino acid solution for standby use.

1 mmol of Rink amide MBHA resin (with a substitution value of about 0.4 mmol/g) was deprotected using 20% PIP/DMF solution for 25 minutes, washed and filtered to obtain a de-Fmoc resin.

The solution containing the activated first protected amino acid was added to the resin without Fmoc, and subjected to coupling reaction for 60 to 300 minutes, filtered and washed to obtain a resin coupling with one protected amino acid.

### (2) Coupling of the 2nd to 39th protected amino acids in the main chain

The corresponding 2nd to 39th protected amino acids were sequentially connected to resin using the same method for connecting the first protected amino acid in the main chain to obtain a resin coupling with 39 amino acids of the main chain.

### (3) Coupling of the first protected amino acid in the side chain

3 mmol of the first protected amino acid of the side chain and 3 mmol of HOBt were dissolved with an appropriate amount of DMF; 3 mmol of DIC was slowly added to the solution of the protected amino acid in DMF under stirring, and the mixture was subjected to reaction at room temperature for 30 min with stirring to obtain a solution of activated protected amino acid.

2.5 mmol of tetrakistriphenylphosphine palladium and 25 mmol of phenylsilane were dissolved in an appropriate amount of dichloromethane, deprotected for 4 hours, filtered and washed to obtain a resin without Alloc for later use.

The solution containing activated first protected amino acid of the side chain was added to the resin without Alloc, subjected to coupling reaction for 60 to 300 minutes, filtered and washed to obtain a resin coupling with the first protected amino acid of the side chain.

### (4) Coupling of protected amino acid of the side chain

The corresponding protected amino acids and single protected fatty acid of the side chain were connected in turn, using the same method for the coupling of the first protected amino acid of the main chain to obtain a peptide resin.

### 2. Production of crude product

The above peptide resin was added with a cleavage reagent with a volume ratio of TFA: water: EDT = 95:5:5 (cleavage reagent 10mL/g resin), stirred evenly, and reacted at room temperature for 3 hours. The reaction mixture was filtered with a sand core funnel, and the filtrate was collected. The resin was washed with a small amount of TFA 3 times, and the filtrates were combined, concentrated under reduced pressure, and added with anhydrous ether for precipitation. Then the precipitate was washed with anhydrous ether 3 times, dried by suction to obtain an off-white powder as the crude product.

### 3. Production of pure product

The above crude product was added with water, stirred, and adjusted to a pH of 8.0 with ammonia water until completely dissolved. The solution was filtered with a mixed membrane filter with a pore size of 0.45µm, and purified for later use.

The purification was performed using high performance liquid chromatography, wherein the packing material of the chromatographic column for purification was reverse phase C18 with a column particle size of 10µm, the mobile phase system was 0.1% TFA/water solution-0.1% TFA/acetonitrile solution, the flow rate of the 30mm×250mm chromatographic column was 20mL/min, a gradient system elution was adopted, and cyclic loading was used for purification. The solution of the crude product was loaded on the chromatographic column, the mobile phase elution was started, and the main peak was collected and evaporated to remove acetonitrile to obtain a purified intermediate concentrate.

The purified intermediate concentrate was filtered with a 0.45 µm filter membrane for standby use. The salt was exchanged by high performance liquid chromatography. The mobile phase system was 1% acetic acid/water solution-acetonitrile, and the packing material of the chromatographic column for purification was reverse phase C18 with a column particle size of 10µm. The flow rate of the 30 mm × 250 mm chromatographic column was 20 mL/min (the flow rate could be adjusted according to the different specifications of the chromatographic column); a gradient elution and cyclic loading method were used, the sample was loaded into the chromatographic column, the mobile phase elution was started, the spectrum was collected, and the change of absorbance was observed. The main peak fraction of salt exchange was collected, and the purity thereof was detected by analytical liquid phase. The main peak fractions of salt exchange were combined, and concentrated under reduced pressure to obtain an aqueous solution of the pure product in acetic acid, which was freeze-dried to obtain 0.35g pure product with a purity of 97.9% and a total yield of 7.0%. The molecular weight was 5012.6 (100% M+H).

### Example 2 Production of Compound 2

The sequence of Compound 2 is set forth in SEQ ID NO: 2:

His-Aib-Gln-Gly-Thr-Phe-Thr-Ser-Asp-Tyr-Ser-Lys-Tyr-Leu-Asp-Glu-Lys-Lys-Ala-Lys(PEG5C
H2CO-γGlu-eicosanedioic acid)-Glu-Phe-Val-Glu-Trp-Leu-Leu-Glu-Gly-Gly-Pro-Ser-Ser-
Gly-Ala-Pro-Pro-Pro-Ser-NH₂

The production method was the same as that of Example 1, and the protected amino acids used are shown in Table 3:

**Table 3 Protected amino acids used in Example 2**

| Order of connecting peptide n= | Protected amino acid |
|---|---|
| 1 | Fmoc-Ser(tBu) |
| 2 | Fmoc-Pro |
| 3 | Fmoc-Pro |
| 4 | Fmoc-Pro |
| 5 | Fmoc-Ala |
| 6 | Fmoc-Gly |
| 7 | Fmoc-Ser(tBu) |
| 8 | Fmoc-Ser(tBu) |
| 9 | Fmoc-Pro |
| 10 | Fmoc-Gly |
| 11 | Fmoc-Gly |
| 12 | Fmoc-Glu(OtBu) |
| 13 | Fmoc-Leu |
| 14 | Fmoc-Leu |
| 15 | Fmoc-Trp(Boc) |
| 16 | Fmoc-Glu(OtBu) |
| 17 | Fmoc-Val |
| 18 | Fmoc-Phe |
| 19 | Fmoc-Glu(OtBu) |
| 20 | Fmoc-Lys(Alloc) |
| 21 | Fmoc-Ala |
| 22 | Fmoc-Lys(Boc) |
| 23 | Fmoc-Lys(Boc) |
| 24 | Fmoc-Glu(OtBu) |
| 25 | Fmoc-Asp(OtBu) |
| 26 | Fmoc-Leu |
| 27 | Fmoc-Tyr(tBu) |
| 28 | Fmoc-Lys(Boc) |
| 29 | Fmoc-Ser(tBu) |
| 30 | Fmoc-Tyr(tBu) |
| 31 | Fmoc-Asp(OtBu) |
| 32 | Fmoc-Ser(tBu) |
| 33 | Fmoc-Thr(tBu) |
| 34 | Boc-Phe |
| 35 | Fmoc-Thr(tBu) |
| 36 | Fmoc-Gly |
| 37 | Fmoc-Gln(Trt) |
| 38 | Fmoc-Aib |
| 39 | Boc-His(Trt) |
| Side chain-1 | Fmoc-PEG₅CH₂COOH |
| Side chain-2 | Fmoc-γGlu-OtBu |
| Side chain-3 | 20-(tert-Butoxy)-20-oxoicosanoic acid |

0.43g of pure product was obtained, with a purity of 97.0% and a total yield of 8.6%. The molecular weight was 4999.6 (100% M+H).

### Example 3 Production of Compound 3

The sequence of Compound 3 is set forth in SEQ ID NO: 3:

His-Aib-Gln-Gly-Thr-Phe-Thr-Ser-Asp-Tyr-Ser-Lys-Tyr-Leu-Asp-Glu-Lys-Lys-Ala-Lys(Gly-Gly
-Ser-Gly-Ser-Gly-γGlu-eicosanedioic acid -γGlu-eicosanedioic acid)-Glu-Phe-Val-Glu-Trp-
Leu-Leu-Glu-Gly-Gly-Pro-Ser-Ser-Gly-Ala-Pro-Pro-Pro-Ser-NH2

The production method was the same as that of Example 1, and the protected amino acids used are shown in Table 4:

**Table 4 Protected amino acids used in Example 3**

| Order of connecting peptide n= | Protected amino acid |
|---|---|
| 1 | Fmoc-Ser(tBu) |
| 2 | Fmoc-Pro |
| 3 | Fmoc-Pro |
| 4 | Fmoc-Pro |
| 5 | Fmoc-Ala |
| 6 | Fmoc-Gly |
| 7 | Fmoc-Ser(tBu) |
| 8 | Fmoc-Ser(tBu) |
| 9 | Fmoc-Pro |
| 10 | Fmoc-Gly |
| 11 | Fmoc-Gly |
| 12 | Fmoc-Glu(OtBu) |
| 13 | Fmoc-Leu |
| 14 | Fmoc-Leu |
| 15 | Fmoc-Trp(Boc) |
| 16 | Fmoc-Glu(OtBu) |
| 17 | Fmoc-Val |
| 18 | Fmoc-Phe |
| 19 | Fmoc-Glu(OtBu) |
| 20 | Fmoc-Lys(Alloc) |
| 21 | Fmoc-Ala |
| 22 | Fmoc-Lys(Boc) |
| 23 | Fmoc-Lys(Boc) |
| 24 | Fmoc-Glu(OtBu) |
| 25 | Fmoc-Asp(OtBu) |
| 26 | Fmoc-Leu |
| 27 | Fmoc-Tyr(tBu) |
| 28 | Fmoc-Lys(Boc) |
| 29 | Fmoc-Ser(tBu) |
| 30 | Fmoc-Tyr(tBu) |
| 31 | Fmoc-Asp(OtBu) |
| 32 | Fmoc-Ser(tBu) |
| 33 | Fmoc-Thr(tBu) |
| 34 | Boc-Phe |
| 35 | Fmoc-Thr(tBu) |
| 36 | Fmoc-Gly |
| 37 | Fmoc-Gln(Trt) |
| 38 | Fmoc-Aib |
| 39 | Boc-His(Trt) |
| Side chain-1 | Fmoc-Gly |
| Side chain-2 | Fmoc-Gly |
| Side chain-3 | Fmoc-Ser(tBu) |
| Side chain-4 | Fmoc-Gly |
| Side chain-5 | Fmoc-Ser(tBu) |
| Side chain-6 | Fmoc-Gly |
| Side chain-7 | Fmoc-γGlu-OtBu |
| Side chain-8 | 20-(tert-Butoxy)-20-oxoicosanoic acid |

0.33 g of pure product was obtained with a purity of 93.4% and a total yield of 6.4%. The molecular weight was 5124.6 (100% M+H).

### Example 4 Production of Compound 4

The sequence of Compound 4 is set forth in SEQ ID NO: 4:

His-Aib-Gln-Gly-Thr-Phe-Thr-Ser-Asp-Tyr-Ser-Lys-Tyr-Leu-Asp-Glu-Lys-Lys-Ala-Lys(Gly-Gly
-Glu-Gly-Glu-Gly-γGlu-eicosanedioic acid)-Glu-Phe-Val-Glu-Trp-Leu-Leu-Glu-Gly-Gly-Gly-
Pro-Ser-Ser-Gly-Ala-Pro-Pro-Pro-Ser-NH2

The production method was the same as that of Example 1, and the protected amino acids used are shown in Table 5:

**Table 5 Protected amino acids used in Example 4**

| Order of connecting peptide n= | Protected amino acid |
|---|---|
| 1 | Fmoc-Ser(tBu) |
| 2 | Fmoc-Pro |
| 3 | Fmoc-Pro |
| 4 | Fmoc-Pro |
| 5 | Fmoc-Ala |
| 6 | Fmoc-Gly |
| 7 | Fmoc-Ser(tBu) |
| 8 | Fmoc-Ser(tBu) |
| 9 | Fmoc-Pro |
| 10 | Fmoc-Gly |
| 11 | Fmoc-Gly |
| 12 | Fmoc-Glu(OtBu) |
| 13 | Fmoc-Leu |
| 14 | Fmoc-Leu |
| 15 | Fmoc-Trp(Boc) |
| 16 | Fmoc-Glu(OtBu) |
| 17 | Fmoc-Val |
| 18 | Fmoc-Phe |
| 19 | Fmoc-Glu(OtBu) |
| 20 | Fmoc-Lys(Alloc) |
| 21 | Fmoc-Ala |
| 22 | Fmoc-Lys(Boc) |
| 23 | Fmoc-Lys(Boc) |
| 24 | Fmoc-Glu(OtBu) |
| 25 | Fmoc-Asp(OtBu) |
| 26 | Fmoc-Leu |
| 27 | Fmoc-Tyr(tBu) |
| 28 | Fmoc-Lys(Boc) |
| 29 | Fmoc-Ser(tBu) |
| 30 | Fmoc-Tyr(tBu) |
| 31 | Fmoc-Asp(OtBu) |
| 32 | Fmoc-Ser(tBu) |
| 33 | Fmoc-Thr(tBu) |
| 34 | Boc-Phe |
| 35 | Fmoc-Thr(tBu) |
| 36 | Fmoc-Gly |
| 37 | Fmoc-Gln(Trt) |
| 38 | Fmoc-Aib |
| 39 | Boc-His(Trt) |
| Side chain-1 | Fmoc-Gly |
| Side chain-2 | Fmoc-Gly |
| Side chain-3 | Fmoc-Glu(OtBu) |
| Side chain-4 | Fmoc-Gly |
| Side chain-5 | Fmoc-Glu(OtBu) |
| Side chain-6 | Fmoc-Gly |
| Side chain-7 | Fmoc-γGlu-OtBu |
| Side chain-8 | 20-(tert-Butoxy)-20-oxoicosanoic acid |

0.30 g of pure product was obtained with a purity of 96.5% and a total yield of 5.8%. The molecular weight was 5208.7 (100% M+H).

### Example 5 Synthesis of control compound

The control compound is OXM3, and its amino acid sequence is set forth in SEQ ID NO: 5:

His-Aib-Gln-Gly-Thr-Phe-Thr-Ser-Asp-Tyr-Ser-Lys-Tyr-Leu-
Asp-Glu-Lys-Lys-Ala-Lys(AEEA-AEEA-γGlu-eicosanedioic acid)-Glu-
Phe-Val-Glu-Trp-Leu-Leu-Glu-Gly-Gly-Pro-Ser-Ser-Gly-NH₂

The production method was the same as in Example 1, and the molecular weight was 4563.1.

### Example 6 Determination of GLP-1 activity

### 1. Determination method

GLP-1R activates the intracellular adenylate cyclase pathway under the stimulation of its specific agonist, so as to increase the cAMP level and ultimately lead to the production and release of insulin. The stable cell line expressing GLP-1R was stimulated by the test substance to rapidly increase the intracellular cAMP level. The relative light units (RLU) in the cells after stimulation by each dose of the test substance were determined by the chemiluminescence method, and then the EC50 of the agonist was calculated. This activity determination method is currently a common determination method for GLP-1 receptor agonist activity at home and abroad.

A CHO-K1 cell line that stably expresses GLP-1R was adopted, and the cells were stimulated with different concentrations of agonists. The relative light units after stimulation of the cells by each dose of agonists were measured. Using OXM3 as a reference substance, the relative biological activity of each agonist was obtained.

### 2. Determination results

The determination results are shown in Table 6.

**Table 6 GLP activity determination**

| **Compound** | **Relative biological activity** |
|---|---|
| Reference | OXM3 |
| Compound 1 | 188.5% |
| Compound 2 | 259.9% |
| Compound 3 | 158.1% |
| Compound 4 | 115.6% |

The test results show that the GLP-1 activity of the new GIP/GLP-I dual agonist analogue is increased by 15% to 159% compared with OXM3.

### Example 7 Gcg activity determination

### 1. Determination method

GcgR activates the intracellular adenylate cyclase pathway under the stimulation of its specific agonist, so as to increase the cAMP level and ultimately lead to the production and release of insulin. The stably cell line expressing GcgR was stimulated by the test substance to rapidly increase the intracellular cAMP level. The relative light units (RLU) in the cells after the stimulation by each dose of agonists were determined by the chemiluminescence method, and then the EC50 of the agonist was calculated. This activity determination method is currently a common determination method for Gcg receptor agonist activity at home and abroad.

A CHO-K1 cell line stably expressing GcgR was adopted. The cells were stimulated with different concentrations of agonists, and the relative light units (RLU) after the stimulation of cells by each dose of agonists were determined. Using OXM3 as a reference, the relative biological activity of the agonist was obtained.

### 2. Determination results

The determination results are shown in Table 7.

**Table 7 Gcg activity determination**

| **Compound** | **Biological activity (%)** |
|---|---|
| Reference | OXM3 |
| Compound 1 | 90.5% |
| Compound 2 | 86.9% |
| Compound 3 | 94.2% |
| Compound 4 | 49.6% |

The determination results show that the Gcg activity of the new GIP/GLP-I dual agonist 1 to 3 is similar to the activity of OXM3.

### Example 8 Determination of preliminary pharmacokinetic properties

The experimental animals were cynomolgus monkeys, and two male cynomolgus monkeys in each group were subcutaneously administrated the compound at a dose of 0.2 mg/kg. The venous blood samples were collected before drug administration (0 h), and 1h, 2h, 3h, 4h, 8h, 12h, 18h, 24h, 48h, 96h, 144h and 168h after administration, respectively. The plasma samples were separated by centrifugation. The blood drug concentrations of the corresponding compounds in the plasma samples were determined by liquid chromatography-mass spectrometry. The half-life of the compound given via subcutaneous (SC) administration is shown in Table 8.

**Table 8 Determination of preliminary pharmacokinetic properties**

| **Compound** | **t_{1/2} (h)** |
|---|---|
| Compound 2 | 140.2 |

The modified compound with the highest biological activity (Compound 2) was detected for its pharmacokinetic properties. The result showed that the half-life of Compound 2 was 140.2 hrs, which was converted to a human half-life of about 280 hrs, much higher than the half-life of OXM3 (120 hrs).

The above is a detailed description of a long-acting dual agonist compound provided by the present invention. Specific examples are used herein to illustrate the principles and embodiments of the present invention. The description of the above examples is only used to help understand the method of the present invention and its core idea. It should be noted that for those skilled in the art, without departing from the principle of the present invention, the present invention can also be improved and modified in several ways, and these improvements and modifications also fall within the scope of protection of the claims of the present invention.

### Sequence Listing

<?xml version="1.0" encoding="UTF-8"?><!DOCTYPE ST26SequenceListing SYSTEM "ST26SequenceListing_V1_3.dtd" PUBLIC "-//WIPO//DTD Sequence Listing 1.3//EN"><ST26SequenceListing productionDate="2023-08-01" softwareVersion="2.1.1" softwareName="WIPO Sequence" fileName="OP230813.xml" dtdVersion="V1_3" nonEnglishFreeTextLanguageCode="zh"
originalFreeTextLanguageCode="zh"><ApplicantFileReference>OP230813</ApplicantFileRefer ence><EarliestPriorityApplicationIdentification><IPOfficeCode>CN</IPOfficeCode><Applicati onNumberText>202210957875.5</ApplicationNumberText><FilingDate>2022-08-10</FilingDa te></EarliestPriorityApplicationIdentification><ApplicantName languageCode="en"> Chengdu Auda Biotechnology Co. , Ltd.</ApplicantName><ApplicantNameLatin>Chengdu Auda Biotechnology Co. , Ltd.</ApplicantNameLatin><InventionTitle languageCode="en"> LONG-ACTING DUAL-AGONIST COMPOUND </InventionTitle><SequenceTotalQuantity>5</SequenceTotalQuantity><SequenceData
sequenceIDNumber="1 "><INSDSeq><INSDSeq_length>39</INSDSeq_length><INSDSeq_mol type>AA</INSDSeq_moltype><INSDSeq_division>PAT</INSDSeq_division><INSDSeq_featu re-table><INSDFeature><INSDFeature_key>source</INSDFeature_key><INSDFeature_locatio n>1..39</INSDFeature_location><INSDFeature_quals><INSDQualifier><INSDQualifier_name >mol_type</INSDQualifier_name><INSDQualifier_value>protein</INSDQualifier_value></IN SDQualifier><IN SDQualifier
id="q2"><INSDQualifier_name>organism</INSDQualifier_name><INSDQualifier_value>synth etic
construct</INSDQualifier_value></INSDQualifier></INSDFeature_quals></INSDFeature><IN SDFeature><INSDFeature_key>MOD_RES</INSDFeature_key><INSDFeature_location>2..20 </INSDFeature_location><INSDFeature_quals><INSDQualifier
id="q3 "><INSDQualifier_name>note</INSDQualifier_name><NonEnglishQualifier_value> X at position 20 is lysine modified with AEEA-AEEA-γGlu-eicosanedioic acid </NonEnglishQualifier_value></INSDQualifier><INSDQualifier
id="q8"><INSDQualifier_name>note</INSDQualifier_name><NonEnglishQualifier _value> X at position 2 is Aib </NonEnglishQualifier _value></INSDQualifier></INSDFeature_quals></INSDFeature></INSD Seq_feature-table><INSDSeq_sequence>HXQGTFTSDYSKYLDEKKAXEFVEWLLEGGPSS GAPPPS</INSDSeq_sequence></INSDSeq></SequenceData><SequenceData
sequenceIDNumber="2"><INSDSeq><INSDSeq_length>39</INSDSeq_length><INSDSeq_mol type>AA</INSDSeq_moltype><INSDSeq_division>PAT</INSDSeq_division><INSDSeq_featu re-table><INSDFeature><INSDFeature_key>source</INSDFeature_key><INSDFeature_locatio n>1..39</INSDFeature_location><INSDFeature_quals><INSDQualifier><INSDQualifier_name >mol_type</INSDQualifier_name><INSDQualifier_value>protein</INSDQualifier_value></IN SDQualifier><IN SDQualifier
id="q5 "><INSDQualifier_name>organism</INSDQualifier_name><INSDQualifier_value>synth etic
construct</INSDQualifier_value></INSDQualifier></INSDFeature_quals></INSDFeature><IN SDFeature><INSDFeature_key>MOD_RES</INSDFeature_key><INSDFeature_location>2..20 </INSDFeature_location><INSDFeature_quals><INSDQualifier
id="q10"><INSDQualifier_name>note</INSDQualifier_name><NonEnglishQualifier_value> X at position 20 is lysine modified with PEG5CH2CO-γGlu-eicosanedioic acid </NonEnglishQualifier _value></INSDQualifier><INSDQualifier
id="q9"><INSDQualifier_name>note</INSDQualifier_name><NonEnglishQualifier _value> X at position 2 is Aib </NonEnglishQualifier _value></INSDQualifier></INSDFeature_quals></INSDFeature></INSD Seq_feature-table><INSDSeq_sequence>HXQGTFTSDYSKYLDEKKAXEFVEWLLEGGPSS GAPPPS</INSDSeq_sequence></INSDSeq></SequenceData><SequenceData
sequencelDNumber="3"><INSDSeq><INSDSeq_length>39</INSDSeq_length><INSDSeq_mol type>AA</INSDSeq_moltype><INSDSeq_division>PAT</INSDSeq_division><INSDSeq_featu re-table><INSDFeature><INSDFeature_key>source</INSDFeature_key><INSDFeature_locatio n>1..39</INSDFeature_location><INSDFeature_quals><INSDQualifier><INSDQualifier_name >mol_type</INSDQualifier_name><INSDQualifier_value>protein</INSDQualifier_value></IN SDQualifier><IN SDQualifier
id="q6"><INSDQualifier_name>organism</INSDQualifier_name><INSDQualifier_value>synth etic
construct</INSDQualifier_value></INSDQualifier></INSDFeature_quals></INSDFeature><IN SDFeature><INSDFeature _key>MOD_RES</INSDFeature_key><INSDFeature_location>2..20 </INSDFeature_location><INSDFeature_quals><INSDQualifier
id="q11 "><INSDQualifier_name>note</INSDQualifier_name><NonEnglishQualifier _value> X at position 2 is Aib </NonEnglishQualifier _value></INSDQualifier><INSDQualifier id="q12"><INSDQualifier _name>note</INSDQualifier_name><NonEnglishQualifier_value> X at position 20 is a lysine modified with Gly-Gly-Ser-Gly-Ser-Gly-γGlu-eicosanedioic acid-γGlu-eicosanedioic acid </NonEnglishQualifier _value></INSDQualifier></INSDFeature_quals></INSDFeature></INSD Seq_feature-table><INSDSeq_sequence>HXQGTFTSDYSKYLDEKKAXEFVEWLLEGGPSS GAPPPS</INSDSeq_sequence></INSDSeq></SequenceData><SequenceData
sequenceIDNumber="4"><INSDSeq><INSDSeq_length>39</INSDSeq_length><INSDSeq_mol type>AA</INSDSeq_moltype><INSDSeq_division>PAT</INSDSeq_division><INSDSeq_featu re-table><INSDFeature><INSDFeature_key>source</INSDFeature_key><INSDFeature_locatio n>1..39</INSDFeature_location><INSDFeature_quals><INSDQualifier><INSDQualifier_name >mol_type</INSDQualifier_name><INSDQualifier_value>protein</INSDQualifier_value></IN SDQualifier><IN SDQualifier
id="q7"><INSDQualifier_name>organism</INSDQualifier_name><INSDQualifier_value>synth etic
construct</INSDQualifier_value></INSDQualifier></INSDFeature_quals></INSDFeature><IN SDFeature><INSDFeature _key>MOD_RES</INSDFeature_key><INSDFeature_location>2..20 </INSDFeature_location><INSDFeature_quals><INSDQualifier
id="q13 "><INSDQualifier _name>note</INSDQualifier_name><NonEnglishQualifier_value> X at position 2 is Aib </NonEnglishQualifier_value></INSDQualifier><INSDQualifier id="q19"><INSDQualifier_name>note</INSDQualifier_name><NonEnglishQualifier_value> X at position 20 is Gly-Gly-Glu-Gly-Glu-Gly-γGlu-eicosanedioic acid-modified lysine</NonEnglishQualifier_value></INSDQualifier></INSDFeature_quals></INSDFeature></ INSDSeq_feature-table><INSDSeq_sequence>HXQGTFTSDYSKYLDEKKAXEFVEWLLEG GPSSGAPPPS</INSDSeq_sequence></INSDSeq></SequenceData><SequenceData
sequenceIDNumber="5"><INSDSeq><INSDSeq_length>34</INSDSeq_length><INSDSeq_mol type>AA</INSDSeq_moltype><INSDSeq_division>PAT</INSDSeq_division><INSDSeq_featu re-table><INSDFeature><INSDFeature_key>source</INSDFeature_key><INSDFeature_locatio n>1..34</INSDFeature location><INSDFeature quals><INSDQualifier><INSDQualifier name >mol_type</INSDQualifier_name><INSDQualifier_value>protein</INSDQualifier_value></IN SDQualifier><IN SDQualifier
id="q21"><INSDQualifier_name>organism</INSDQualifier_name><INSDQualifier_value>synt hetic
construct</INSDQualifier_value></INSDQualifier></INSDFeature_quals></INSDFeature><IN SDFeature><INSDFeature _key>MOD_RES</INSDFeature_key><INSDFeature_location>2..20 </INSDFeature_location><INSDFeature_quals><INSDQualifier
id="q23 "><INSDQualifier _name>note</INSDQualifier_name><NonEnglishQualifier_value> X at position 20 is lysine modified with AEEA-AEEA-γGlu-eicosanedioic acid </NonEnglishQualifier _value></INSDQualifier><INSDQualifier
id="q22"><INSDQualifier _name>note</INSDQualifier_name><NonEnglishQualifier_value> X at position 2 is Aib </NonEnglishQualifier _value></INSDQualifier></INSDFeature_quals></INSDFeature></INSD Seq_feature-table><INSDSeq_sequence>HXQGTFTSDYSKYLDEKKAXEFVEWLLEGGPSS G</INSDSeq_sequence></INSDSeq></SequenceData></ST26SequenceListing>

## Claims

1. A compound, comprising:
(I) an amino acid sequence set forth in Formula I,
His-AA1-Gln-Gly-Thr-Phe-Thr-Ser-Asp-Tyr-Ser-Lys-Tyr-Leu-Asp-Glu-Lys-Lys-Ala-Lys(R)-Glu -Phe-Val-Glu-Trp-Leu-Leu-AA2-Gly-Gly-Pro-Ser-Ser-Gly-Ala-Pro-Pro-Pro-Ser-AA3 Formula I
AA1 in Formula I is selected from the group consisting of Aib, Acpr, Acp, Acpe and Ach;
AA2 in Formula I is selected from the group consisting of Glu and Ser;
AA3 in Formula I is selected from the group consisting of NH₂ and OH;
R in Formula I is selected from the group consisting of HO₂C(CH₂)ₙ₁CO-(AA4)ₙ₂-(PEGₙ₃(CH2)ₙ₄CO)ₙ₅- and HO₂C(CH₂)ₙ₁CO-(AA4)ₙ₂-(AA5)ₙ₆-;
wherein, AA4 is selected from the group consisting of γGlu, εLys, β-Ala, γ-aminobutyric acid and 5-Ava; and
AA5 is selected from the group consisting of Gly, Ser, Thr, Asp, Glu, Aad, Lys, Orn, Dab and Dap; or
(II) an amino acid sequence obtained from substitution, deletion, addition and/or replacement of one or more amino acids of the amino acid sequence set forth in (I) ; or
(III) a sequence with more than 90% homology to the amino acid sequence set forth in (I); or
(IV) a pharmaceutically acceptable salt, solvate, chelate or non-covalent complex of the compound shown in Formula I; and/or
(V) a drug precursor based on the compound shown in Formula I; and/or
(VI) a mixture comprising (I), (II), (III), (IV) and/or (V).

2. The compound according to claim 1, wherein:
n1 is an integer selected from 10 to 20;
n2 is an integer selected from 1 to 5;
n3 is an integer selected from 1 to 30;
n4 is an integer selected from 1 to 5;
n5 is an integer selected from 1 to 10; and
n6 is an integer selected from 1 to 10.

3. A method for producing the compound according to claim 1 or 2, comprising:
Step 1, performing solid-phase polypeptide synthesis to obtain a peptide resin; and
Step 2, performing acid hydrolysis and purification to obtain the compound.

4. Use of any one of the following in the manufacture of a GLP-1 and Gcg dual agonist:
(I) the compound according to claim 1 or 2; and/or
(II) the compound produced by the method according to claim 3.

5. Use of any one of the following in the manufacture of a medicament for preventing and/or treating a disease:
(I) the compound according to claim 1 or 2; and/or
(II) the compound produced by the method according to claim 3.

6. The use according to claim 5, wherein the disease includes:
type II diabetes, impaired glucose tolerance, type I diabetes, obesity, hypertension, metabolic syndrome, dyslipidemia, cognitive impairment, atherosclerosis, myocardial infarction, coronary heart disease, cardiovascular disease, stroke, inflammatory bowel syndrome and/or dyspepsia or gastric ulcer, liver fibrosis and/or pulmonary fibrosis.

7. Use of any one of the following in the manufacture of a drug and/or drug combination for treating type II diabetes with long-lasting efficacy and/or preventing deterioration of type II diabetes:
(I) the compound according to claim 1 or 2; and/or
(II) the compound produced by the method according to claim 3.

8. Use of the compound according to claim 1 or 2, or the compound produced by the method according to claim 3 in the manufacture of a drug and/or drug combination for regulating blood sugar in the body,
wherein, the regulation of blood sugar in the body includes reducing food intake, reducing β cell apoptosis, increasing pancreatic β cell function, increasing β-cell mass and/or restoring the sensitivity of β-cell to glucose.

9. A drug or drug combination, comprising any one of the following:
(I) the compound according to claim 1 or 2; and/or
(II) the compound produced by the method according to claim 3.

10. A method for regulating blood sugar in the body, comprising administering any one of the following to a subject in need thereof:
(I) the compound according to claim 1 or 2; and/or
(II) the compound produced by the method according to claim 3; and/or
(III) the drug or drug combination according to claim 9.
